# FASCICULE DE BREVET EUROPEEN

(11) **EP 0 684 802 B1**
(45) Date de publication et mention de la délivrance du brevet: **29.01.1997**
(21) Numéro de dépôt: 94906951.2
(22) Date de dépôt: 16.02.1994
(51) Int. Cl.: A61F 2/04

(54) **PROTHESE DESTINEE AU TRAITEMENT D'UNE LUMIERE OU VOIE NATURELLE, NOTAMMENT PROTHESE ENDO-URETHRALE**
PROTHESE ZUR BEHANDLUNG EINES LUMENS ODER EINES NATÜRLICHEN KANALS, INSBESONDERE URETHRALE ENDOPROTHESE
PROSTHESIS FOR TREATING A NATURAL CHANNEL OR LUMEN, PARTICULARLY AN ENDO-URETHRAL PROSTHESIS

(30) Priorité: 19.02.1993 FR 9302284
(43) Date de publication de la demande: 06.12.1995
(73) Titulaire: Devonec, Marian, F-01700 Miribel (FR)
(72) Inventeur: Devonec, Marian, F-01700 Miribel (FR)
(74) Mandataire: Maureau, Philippe
(86) Numéro de dépôt international: FR9400171
(87) Numéro de publication internationale: WO9418907

(56) Documents cités:
- WO-A-91/16005
- FR-A- 2 667 783
- US-A- 3 657 744
- US-A- 4 973 301
- US-A- 5 122 154

## Description

La présente invention concerne le traitement de lumières ou voies naturelles du corps humain ou animal, par lesquelles s'effectue un transit ou un écoulement d'un fluide, notamment d'un fluide corporel, liquide ou gazeux, de part et d'autre d'un-sphincter. Les voies urinaires, aéro-digestives, gynécologiques constituent des lumières naturelles au sens de la présente invention.

Par "traitement", on entend aussi bien une intervention de type mécanique, visant à rétablir un écoulement, au préalable perturbé ou empêché, en raison d'une obstruction ou d'une sténose de la lumière naturelle, qu'un traitement ou intervention à visée thérapeutique, par exemple pour guider la cicatrisation de la paroi de la lumière naturelle, après une intervention chirurgicale, ou pour réduire une hyperplasie d'un organe ou glande entourant cette même lumière.

La présente invention sera introduite, définie, et décrite, à titre d'exemple non limitatif, par référence aux prothèses endo-uréthrales qui sont mises en oeuvre dans l'urèthre, en relation avec le sphincter strié.

Conformément au document FR-A-2 667 783, on a décrit une prothèse endo-uréthrale, constituée par un élément tubulaire, de forme générale cylindrique, dont la paroi comprend un matériau biocompatible relativement lisse et mou, par exemple un caoutchouc siliconé, au moins dans sa partie externe. Cet élément tubulaire est suffisamment souple pour se conformer au profil anatomique de l'urèthre et à ses mouvements, mais suffisamment rigide, notamment en direction radiale ou diamétrale, pour maintenir un passage artificiel dans l'urèthre. Cet élément tubulaire est destiné à être placé dans l'urèthre, sans passer au travers du sphincter strié, dans le segment prostatique et/ou dans l'un quelconque des segments membraneux, bulbaire, périnéal et pénien.

Par "élément tubulaire", on entend tout élément dont la surface extérieure est décrite par une génératrice correspondant à une ligne droite ou courbe, ou autre, autour d'un axe.

Selon le document FR-A-2 667 783, le maintien de l'élément tubulaire dans l'urèthre est obtenu, principalement par l'appui élastique en extension radiale de la paroi dudit élément contre la paroi uréthrale, et secondairement par différentes entailles ménagées dans la paroi du même élément tubulaire, dégageant des becs d'accrochage avec la paroi uréthrale, un peu à la manière d'écailles.

En pratique, une telle prothèse n'est pas autostatique, pour différentes raisons :
- l'appui élastique de l'élément tubulaire est insuffisant pour le maintenir en position dans l'urèthre, sauf à prévoir ou obtenir un appui très important, susceptible d'endommager la paroi uréthrale, conduisant à des douleurs du patient, et rendant de toute façon l'extraction de la prothèse implantée, difficile ou impossible
- les becs d'accrochage, ou écailles, ne coopèrent avec la paroi uréthrale, selon sa direction longitudinale, que dans un seul sens correspondant à l'interdiction ou la limitation de la descente de la prothèse ; ces becs d'accrochage ne peuvent donc empêcher la prothèse de remonter
- la nature extérieurement lisse de l'élément tubulaire favorise son glissement naturel contre la paroi uréthrale, en particulier lors des différents mouvements de l'urèthre
- et enfin, les différentes entailles constituent autant de surfaces ponctuelles d'appui, ou zones de turbulence, sur lesquelles s'exercent tant la pression statique que la pression dynamique du flux urinaire, lors de la miction ; ceci conduit à favoriser la descente de la prothèse uréthrale.

Conformément au document WO91/16005, on connaît par ailleurs des prothèses métalliques consistant en deux éléments, constitués chacun par des spires métalliques jointives ou non, destinés à être disposés dans l'urèthre respectivement de part et d'autre du sphincter strié. Pour chaque élément, ces spires compressibles de manière centripète et expansibles de manière centrifuge, déterminent dans leur position expansée une surface d'enveloppe de section variable d'une extrémité à l'autre.

On connaît aussi des prothèses constituées par un tube métallique ou non, ajouré, expansible au moment de son implantation dans l'urèthre.

Aujourd'hui, les différentes prothèses uréthrales proposées ou décrites, n'ont pas su concilier :
- d'un côté, l'autostatisme qui suppose selon les solutions précédentes, d'une manière ou d'une autre, un certain ancrage de la prothèse sur la muqueuse de la paroi uréthrale
- et de l'autre côté, une facilité d'introduction et surtout d'extraction de la prothèse, c'est-à-dire sa réversibilité.

La présente invention a pour objet une prothèse, et notamment une prothèse endo-uréthrale, autostatique, pouvant être introduite et extraite de manière atraumatique de la lumière ou voie naturelle, dans laquelle elle est implantée.

Une prothèse selon l'invention comprend deux éléments tubulaires, obtenus à partir d'un matériau biocompatible relativement lisse et mou tel qu'un caoutchouc silicone, destinés à être disposés dans la lumière, respectivement de part et d'autre du sphincter, et attachés l'un à l'autre par un moyen de liaison, souple et déformable, destiné à être pris dans l'orifice du sphincter. Chaque élément présente une section extérieure sensiblement constante d'une extrémité à l'autre dudit élément.

Préférentiellement, ce moyen de liaison consiste en un manchon souple, dont les deux extrémités sont reliées en continuité d'écoulement respectivement avec les deux éléments tubulaires ; ce moyen de liaison peut être aussi un simple fil ou plusieurs fils de liaison, attachés individuellement à leurs deux extrémités, respectivement aux deux éléments tubulaires.

Une prothèse comportant les caractéristiques techniques définies précédemment, apporte par ailleurs les avantages déterminants suivants.

Le manchon souple ou moyen de liaison constitue une zone prédéterminée de plicature de la prothèse, qui est susceptible d'absorber sans raideur tous les mouvements de la lumière naturelle. Cette zone prédéterminée de plicature permet le travail harmonieux du sphincter. La longueur de ce manchon souple est par ailleurs adaptée à celle du sphincter.

Une telle prothèse présente un profil extérieur régulier, ce qui la rend atraumatique, aussi bien lors de son insertion que de son retrait, lesquels peuvent être faits sans anesthésie générale, et ce qui apporte de l'aisance quant à son positionnement. Ceci la rend aussi biocompatible, au sens où elle n'irrite pas la muqueuse interne de la lumière ou voie naturelle.

Une telle prothèse présente aussi une surface intérieure continue, régulière, en particulier quant à sa section interne, ce qui lui confère de très bonnes propriétés hydrauliques, c'est-à-dire sans obstacle, par exemple vis-à-vis d'un écoulement urinaire. S'agissant de la miction, une prothèse uréthrale selon l'invention n'est pas mobilisée par le flux urinaire.

Une telle prothèse est auto-statique par rapport au sphincter : au repos, le sphincter l'immobilise, et en miction son glissement est empêché par les deux éléments tubulaires, respectivement en butée de part et d'autre du sphincter.

Une telle prothèse est aussi particulièrement facile à mettre en place, avec des instruments simples, en particulier sans recourir nécessairement à des contrôles endoscopiques, ou radiologiques. En particulier, comme exposé ci-après, par simple glissement de la prothèse endo-uréthrale le long de l'urèthre, celle-ci s'immobilise automatiquement en bonne position, au moment où le manchon souple arrive au niveau du sphincter qui se referme sur lui.

De manière essentielle, une telle prothèse ne gène ou ne perturbe les fonctions du sphincter ; elle s'ouvre et se ferme, et travaille donc de façon symétrique et étanche, sous l'action du sphincter.

La présente invention est maintenant décrite par référence aux dessins annexés, dans lesquels :
- la figure 1 représente une prothèse endo-uréthrale conforme à un premier mode d'exécution de l'invention ;
- la figure 2 représente une coupe anatomique des voies urinaires du corps humain masculin ; une prothèse selon figure 1 est représentée sur cette coupe, dans sa position implantée ;
- les figures 3 et 4 représentent de manière schématique, la coopération d'une prothèse selon l'invention et du sphincter strié, respectivement en écoulement par le relâchement du sphincter, et en obturation par la contraction du sphincter ;
- la figure 5 représente trois variantes, référencées (a) à (c) d'un manchon souple appartenant à une prothèse selon figure 1 ; chaque variante (a) à (c) est représentée respectivement en position d'écoulement (miction), en position d'obturation (continence), et en coupe transversale ;
- la figure 6 représente une prothèse endo-uréthrale conforme à un deuxième mode d'exécution de l'invention ;
- la figure 7 représente une prothèse endo-uréthrale conforme à un troisième mode d'exécution de l'invention ;
- la figure 8 représente un ensemble pour l'insertion d'une prothèse selon figure 6, cet ensemble étant montré en haut selon la figure 8b en position démontée avec la prothèse, et en bas selon la figure 8a en position montée avec la prothèse ;
- les figures 9 à 12 représentent respectivement quatre étapes successives de la procédure d'insertion d'une prothèse selon la figure 6 ;
- la figure 13 représente une prothèse conforme à un dernier mode d'exécution de l'invention.

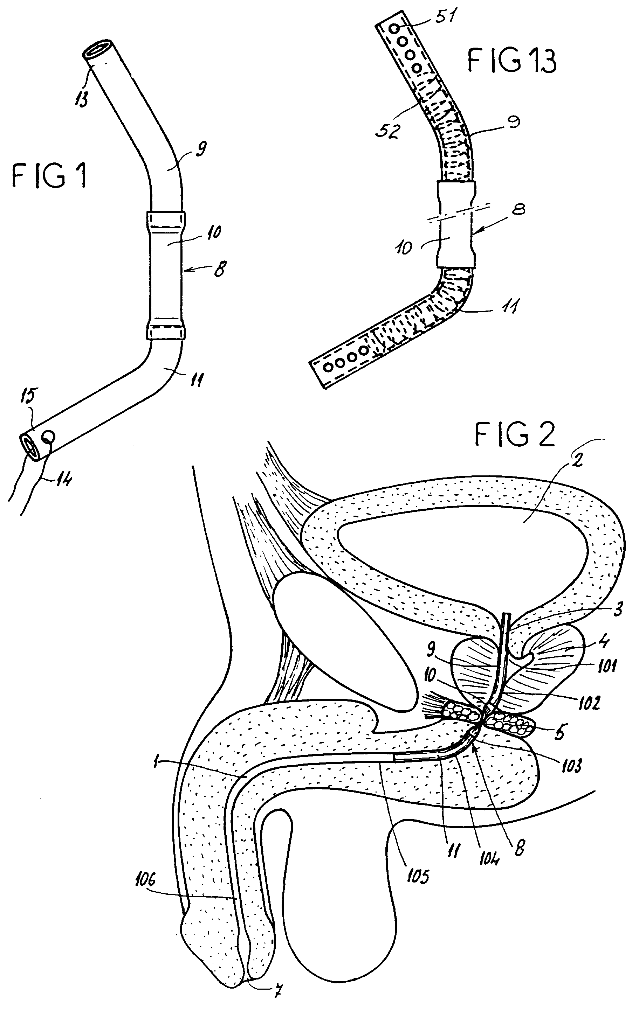

Conformément à la figure 2, l'urèthre 1 s'étend de bas en haut, à partir du méat urinaire 7 jusqu'au col vésical 3 de la vessie 2. Au-dessus du sphincter strié 5, l'urèthre comprend un segment prostatique sus-montanal 101, et un segment prostatique sous-montanal 102, de part et d'autre du verumontanum. Au-dessous du sphincter 5, l'urèthre comprend vers le méat 7, le segment membraneux 103, le segment bulbaire 104, le segment périnéal 105, et enfin le segment pénien 106.

Une prothèse 8 selon l'invention, telle que représentée à la figure 1, est destinée à être implantée comme décrit ci-après, dans l'urèthre 1, de part et d'autre du sphincter 5.

Ex vivo, c'est-à-dire dans sa conformation non implantée, représentée à la figure 1, une prothèse 8 selon l'invention comprend deux éléments tubulaires 9 et 11, de forme cylindrique dans la représentation de la figure 1, dont la paroi est constituée par un matériau biocompatible, éventuellement biodégradable, relativement lisse et mou, par exemple un caoutchouc silicone. Chaque élément tubulaire 9 ou 11 est suffisamment souple, pour se conformer au segment de l'urèthre dans lequel il doit être placé, tout en étant suffisamment rigide pour maintenir un passage artificiel endo-uréthral. Chaque élément tubulaire 9 ou 11 présente une section extérieure sensiblement constante, d'une extrémité à l'autre dudit élément. Ces deux éléments tubulaires sont reliés ou attachés l'un à l'autre, par un moyen de liaison, consistant selon les modes d'exécution des figures 1, 6, 7 et 13, en un manchon souple 10, destiné à être pris dans l'orifice du sphincter 5, comme montré une fois implanté à la figure 2.

Comme représenté à la figure 2, la prothèse mise en place, décrite ci-après, comprend donc de manière continue, les deux éléments tubulaires 9 et 11 disposés dans l'urèthre 1, respectivement de part et d'autre du sphincter 5, et attachés l'un à l'autre par le manchon souple 10, pris dans l'orifice du sphincter 5.

Comme montré par la figure 1, les deux extrémités du manchon souple 10 sont reliées en continuité d'écoulement, respectivement avec les deux éléments tubulaires 9 et 11. La paroi du manchon 10 est plus souple, notamment plus mince que la paroi de chaque élément tubulaire 9 ou 11. En pratique, cette paroi du manchon 10 peut être obtenue à partir d'un voile de silicone, les éléments tubulaires 9 et 11 étant constitués, en ce qui les concerne, par une paroi de caoutchouc silicone, relativement plus épaisse. La liaison entre le manchon 10 d'une part, et les deux éléments tubulaires 9 et 11, est obtenue de toute façon appropriée, par exemple par collage. De manière représentée à la figure 5, le manchon souple 10 peut être ajouré de manière distribuée selon son pourtour, notamment par des fentes ou fenêtres longitudinales, de manière à faciliter la plicature du manchon 10, et à assurer la continence ; les dimensions de ces fenêtres ou fentes sont telles que la liaison entre les eléments tubulaires 9 et 11 est réduite à un faisceau de bandelettes ou de fils, dans le prolongement desdits éléments tubulaires 9 et 11.

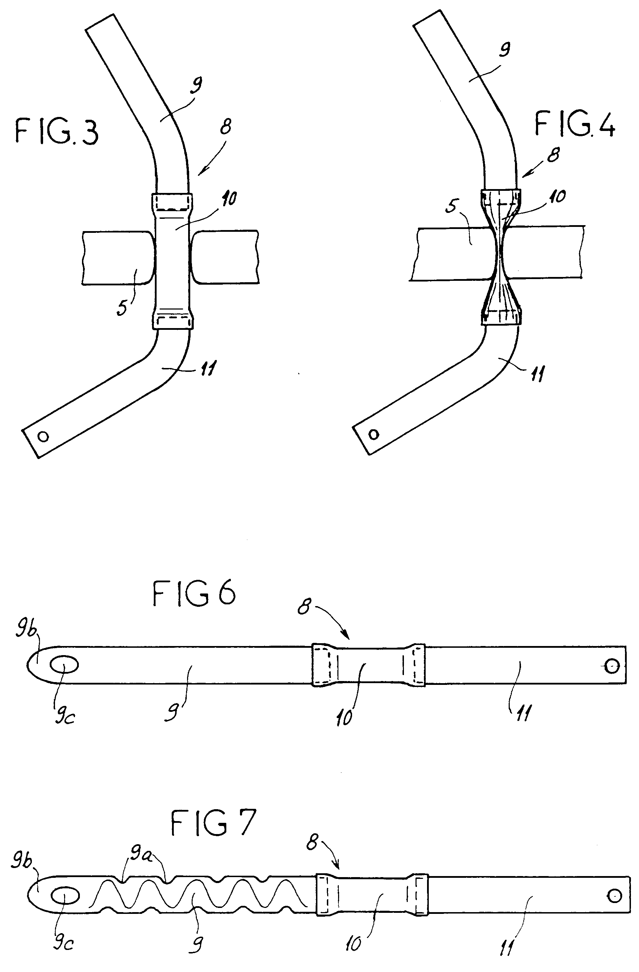

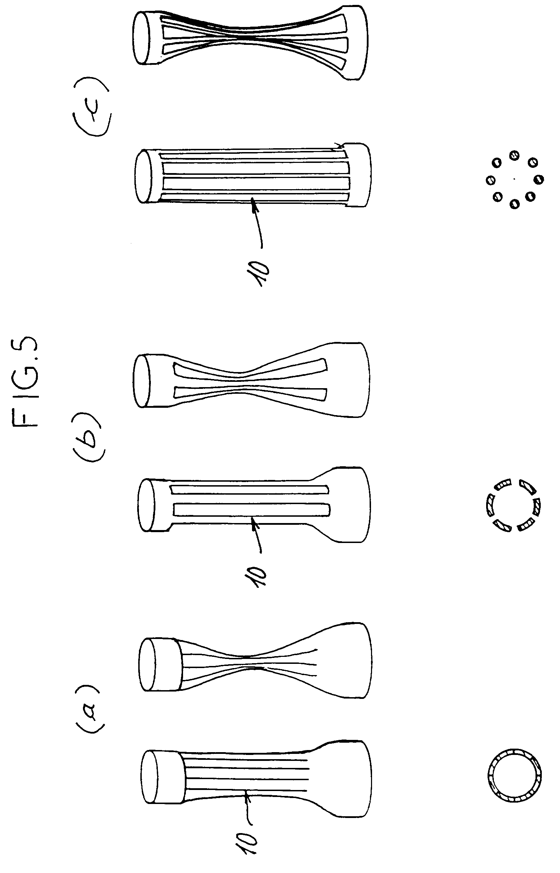

Comme montré par les figures 3 et 4 et de manière schématique, la paroi du manchon souple 10 est susceptible de prendre deux conformations sous l'action du sphincter strié 5, à savoir une conformation d'écoulement représentée à la figure 3, ayant la forme d'un cylindre, par le relâchement du sphincter 5, et une conformation d'obturation, représentée à la figure 4, correspondant à une forme biconique ou en sablier, par la contraction du sphincter 5.

Comme représenté à la figure 3, mais également dans sa conformation ex vivo, les éléments tubulaires 9 et 11 forment avec le manchon souple 10, un conduit de section intérieure sensiblement constante selon la direction longitudinale de la prothèse 8. Chaque élément tubulaire 9 ou 11 a un profil extérieur sensiblement constant ou régulier selon la même direction longitudinale. Quelle que soit la conformation, les deux extrémités du manchon souple 10 sont reliées en continuité d'écoulement, respectivement avec les deux éléments tubulaires 9 et 11. Ceci étant, de manière non représentée, lorsque le calibre du méat uréthral le permet, l'élément tubulaire inférieur 11 peut avoir une section extérieure plus importante que celle de l'élément tubulaire supérieur 9, dans le but de favoriser l'autostatisme, et de prévenir la migration de la prothèse vers le haut.

Comme représenté à la figure 1, ex vivo, c'est-à-dire en dehors de toute contrainte imposée par son implantation dans l'urèthre, chaque élément tubulaire 9 ou 11 peut présenter une angulation prédéterminée selon son axe, adaptée, identique ou différente de l'angulation naturelle ou physiologique du segment dans lequel ledit élément tubulaire est destiné à être implanté. Plus précisément, en considérant la direction et le sens d'implantation selon la figure 2, le segment tubulaire supérieur 9 peut présenter une angulation comprise entre 140 et 160°, et de préférence égale à 150°, et le segment tubulaire inférieur 11 peut présenter une angulation comprise entre 110 et 130°, et de préférence égale à 120°. Cette angulation est rémanente, ce qui veut dire que sous contrainte, elle peut être supprimée, mais que hors contrainte, chaque élément tubulaire reprend, de manière élastique, sensiblement son angulation d'origine. L'angulation retenue, par exemple celle de l'élément tubulaire inférieur 11 dans sa partie adjacente au manchon 10 en regard du sphincter 5, peut être choisie pour s'opposer à la forme anatomique de l'urèthre, par exemple du segment bulbaire 104, et ainsi s'opposer à la migration de la prothèse vers le haut, au travers du sphincter 5.

Le diamètre extérieur des deux éléments tubulaires est voisin de 7,3 mm (22 selon la codification Charrière) ; il peut être de 8 mm (Charrière 24) pour l'élément tubulaire inférieur.

Comme montré à la figure 1, la prothèse 8 comprend un fil d'extraction 14 à son extrémité inférieure 15, toujours selon le sens d'implantation représenté à la figure 2.

Conformément au mode d'exécution représenté à la figure 13, chaque élément tubulaire 9 ou 11 peut être ajouré par des perforations 51, distribuées selon sa longueur, du côté de son extrémité libre, c'est-à-dire opposé au manchon 10 ; ces perforations favorisent l'incrustation de la prothèse vis-à-vis de la paroi uréthrale.

Conformément au mode d'exécution représenté à la figure 7, l'élément tubulaire supérieur 9 présente une ou des encoches distribuées selon sa longueur, par exemple une encoche 9a en hélice, à la manière d'un filet de vis, ou des encoches disposées en quinconce, et ceci toujours pour favoriser l'élimination des sécrétions naturelles de la prostate ou de débris tissulaires libérés par son traitement.

Comme montré par la figure 13, une spirale 52 métallique ou non, constitue une armature de chaque élément tubulaire 9 ou 11. Elle est noyée dans le matériau élastomère du tube de chaque élément tubulaire, comme représenté à la figure 13, mais elle peut être également apparente extérieurement, par exemple à des fins de traitement thérapeutique de la paroi uréthrale. L'armature 52 de chaque élément tubulaire 9 ou 11 s'étend à partir du manchon 10, sur une partie seulement de la longueur dudit élément tubulaire, de telle sorte que sa partie restante demeure sécable, par exemple pour adapter la longueur de l'élément tubulaire 9 à celle du segment prostatique 101/102, ou pour adapter la longueur de l'élément tubulaire 11, par rapport à la localisation d'une sténose dans la partie de l'urèthre 1 sous le sphincter 5.

Comme le montre la figure 13, le matériau élastomère recouvre, au moins vers l'extérieur l'armature métallique 52, et constitue donc au moins la partie externe de chaque élément tubulaire 9 ou 11, au contact de la paroi uréthrale.

Conformément aux figures 6 et 7, l'extrémité supérieure de l'élément tubulaire supérieur 9, c'est-à-dire celle opposée au manchon 10 de liaison, est transversalement obturée, et se présente sous la forme d'un bout 9b convexe et arrondi. Ce bout présente un ou deux orifices latéraux 9c de communication avec l'intérieur de l'élément tubulaire 9.

Chaque élément tubulaire 9 ou 11 peut être revêtu sur sa surface extérieure, d'un produit thérapeutique, aux fins d'un traitement de l'urèthre.

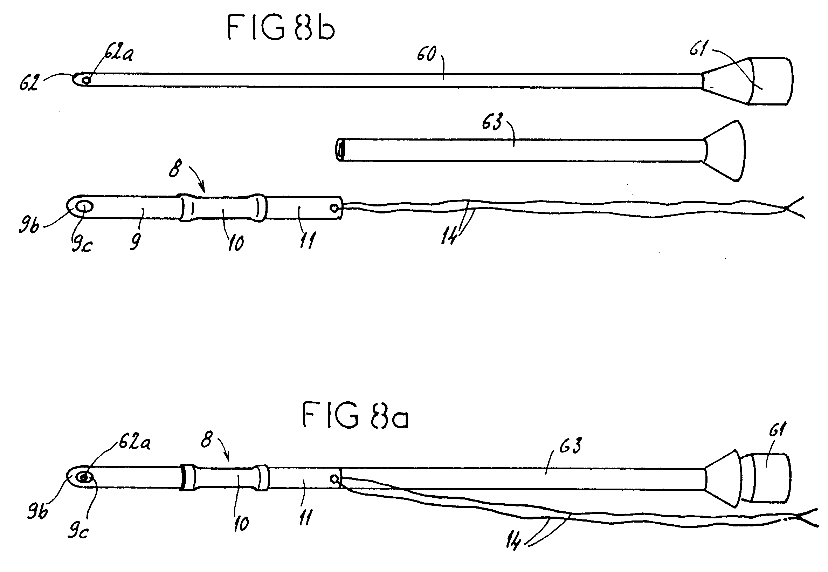

Conformément à la figure 8b, pour insérer une prothèse uréthrale conforme à la figure 6 ou à la figure 7, on dispose d'un ensemble comprenant :
- un mandrin 60 semi-rigide, creux dont la section extérieure est adaptée pour recevoir par emmanchement, la prothèse 8 ; ce mandrin présente, d'un côté une butée extérieure 61 conique, et de l'autre côté, une extrémité 62 fermée, contre laquelle l'extrémité obturée 9b de la prothèse 8 vient en butée, dans sa position emmanchée ; cette extrémité fermée 62 du mandrin 60 présente un oeil d'écoulement 62a à l'intérieur du mandrin 60, pouvant venir en coïncidence avec l'orifice latéral 9c de la prothèse, toujours dans sa position emmanchée ;
- un poussoir rigide creux 63, dont la section intérieure est adaptée pour un emmanchement dudit poussoir sur le mandrin semi-rigide 60 ; la longueur du poussoir 63 est adaptée pour servir, dans sa position emmanchée, d'entretoise entre la prothèse emmanchée 8 et la butée extérieure 61 du mandrin 60.

Le dispositif d'insertion décrit précédemment est utilisé de la manière suivante.

On assemble en une seule pièce :
- le mandrin 60 ;
- le poussoir 63, emmanché sur le mandrin 60, en venant en butée contre son extrémité proximale, matérialisée par la butée conique 61 ;
- et enfin, la prothèse 8, également emmanchée sur l'extrémité libre du mandrin 60, pour venir en butée par son extrémité 9b contre l'extrémité libre 62 du mandrin 60.

Dans cette position assemblée, représentée au bas de la figure 8a, le poussoir 63 sert d'entretoise entre la prothèse 8 et la butée extérieure 61 du mandrin 60. La prothèse 8 est orientée angulairement par rapport au mandrin 60, de telle manière que l'oeil d'écoulement 62a est en coïncidence avec l'orifice latéral 9c de la prothèse, de section plus importante.

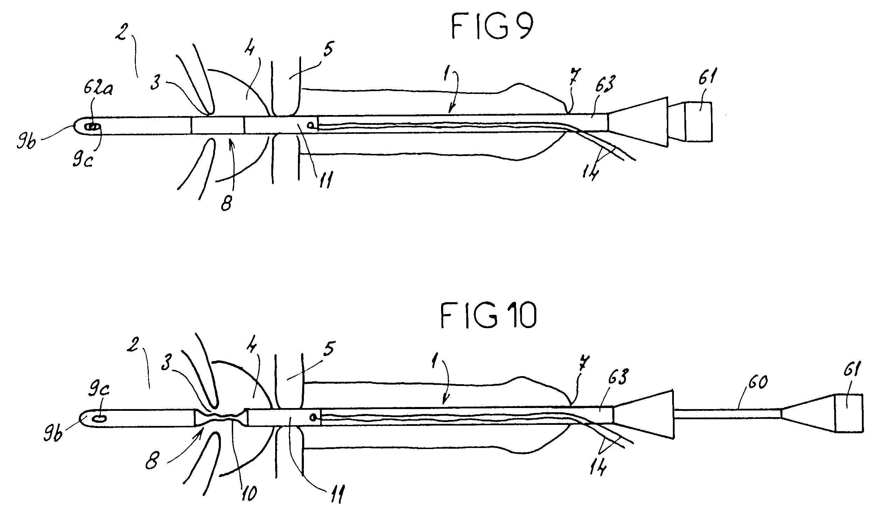

Après lubrification du canal 1 de l'urèthre, l'ensemble assemblé est inséré à travers le méat 7 uréthral, jusqu'à ce que l'extrémité supérieure 9b de la prothèse débouche dans la vessie 2. Dès ce moment, l'écoulement d'urine par le mandrin 60 signale l'arrivée du dispositif d'insertion dans la vessie, conformément à la position représentée à la figure 9.

Le mandrin 60 est alors libéré et retiré, tout en maintenant le poussoir 63 en position dans l'urèthre, de façon à ce que la prothèse 8 ne bouge pas ; conférer figure 10.

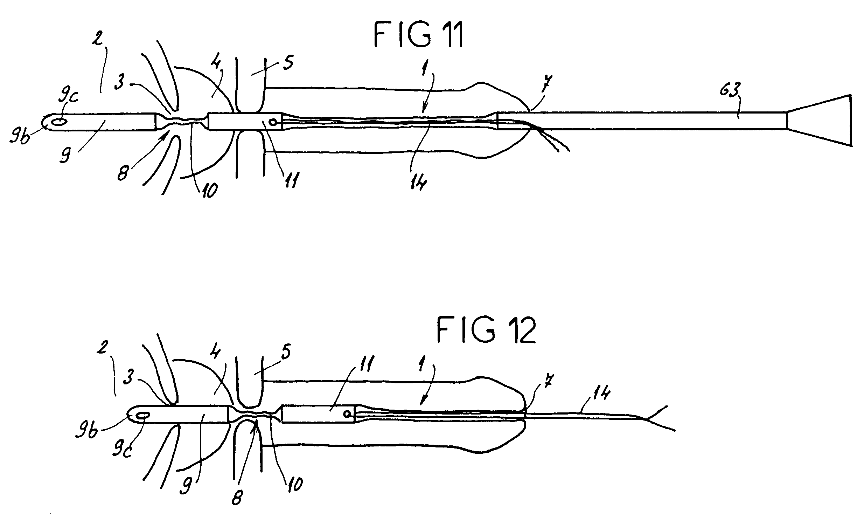

Une fois le mandrin 60 retiré, le poussoir 63 est lui-même retiré de l'urèthre 1 ; conférer figure 11. A ce stade, l'élément tubulaire inférieur 11 de la prothèse 8 se trouve à la hauteur du sphincter strie.

Une traction douce vers le bas, exercée par le fil 14, permet de faire glisser l'élément tubulaire inférieur 11 et d'engager le manchon souple 10 dans le sphincter.

Dès ce moment, l'opérateur ressent immédiatement un blocage en extraction, qui correspond au bon positionnement de la prothèse 8 par rapport au sphincter 5, qui se ferme sur le manchon souple 10. La position correcte de la prothèse est donc automatiquement trouvée ; conférer figure 12.

Pendant cette dernière opération, l'opérateur peut également s'aider de son index en réalisant un toucher rectal, car l'élément tubulaire inférieur 11 est bien perçu au doigt, et son passage en deça du sphincter 5 est bien noté, avec la disparition de la consistance dudit élément tubulaire inférieur, remplacé par le manchon souple 10. Le positionnement correct de la prothèse 8, par rapport au sphincter 5, est totalement indolore, et il ne nécessite aucune instrumentation rigide, par exemple une pince. Il ne nécessite aussi aucun contrôle radiologique. Tout au plus, peut-il être facilité par l'utilisation d'une sonde d'échographie, qui remplacera le doigt au niveau du rectum.

A partir de la figure 12, la prothèse mise en place peut être facilement retirée, à l'aide d'une pince par exemple, par une simple traction sur le bord libre de l'élément tubulaire inférieur 11, la résistance du sphincter 5 est faible, puisque la pression de clôture de ce dernier est inférieure à la pression exercée par une colonne d'eau de 100 cm. Dès que l'élément supérieur 9 est engagé dans le sphincter 5, le glissement et le retrait de la prothèse 8 se font sans aucun accrochage par rapport à la paroi uréthrale.

Une prothèse conforme à la figure 6, mise en place avec un dispositif d'insertion selon la figure 8, à la fois est atraumatique, se positionne de manière quasi-automatique par rapport au sphincter, et est autostatique.

Cette prothèse est atraumatique, à la fois par sa configuration extérieure et par sa manipulation :
- sa configuration est atraumatique, en raison de son extrémité supérieure 9b convexe ou arrondie, éventuellement flexible, ce qui évite tout traumatisme de la muqueuse uréthrale, et donc tout saignement, en raison de sa surface extérieure quasiment lisse, c'est-à-dire sans aspérité ou relief particulier, et en raison du manchon intermédiaire souple, permettant le jeu normal du sphincter ;
- sa manipulation est atraumatique, car, comme décrit précédemment, sa mise en place est aussi douce et simple que celle d'une sonde à demeure ; en permettant le fonctionnement normal du sphincter, la prothèse se présente à la manière d'une sonde à demeure continente ; pendant toute la durée où le patient porte cette prothèse, la vessie n'est pas blessée, ce qui évite toute formation de caillots ; et au moment de son retrait, la prothèse 8 glisse d'un seul bloc, sans générer de douleur particulière pour le patient.

Le positionnement d'une prothèse selon l'invention est quasi automatique, puisqu'en définitive, il est assuré par la simple perception tactile d'une résistance, au moment où le sphincter se ferme sur le manchon souple 10. Selon l'invention, le positionnement repose sur un repérage tactile, à la fois par la traction exercée sur le fil 14, et/ou par un toucher rectal qui permet de sentir la fin du franchissement du sphincter 5 par l'élément tubulaire inférieur 11. Et selon l'invention, tout défaut de mise en position est récupérable, en mobilisant la prothèse par une traction faible générant peu de douleur pour le patient, et ne requérant pas de ce fait, une anesthésie générale.

Et enfin, la prothèse est autostatique, par la présence des deux éléments tubulaires 9 et 11 de part et d'autre du sphincter 5. Cet autostatisme peut d'ailleurs être amélioré, par la présence d'orifices ou d'encoches, comme décrit aux figures 7 et 13, dans lesquelles s'engage la muqueuse uréthrale.

## Revendications

1. Prothèse (8) destinée au traitement d'une lumière (1) ou voie naturelle d'un corps humain ou animal, par laquelle s'effectue un écoulement de part et d'autre d'un sphincter (5), ladite prothèse comprenant un élément tubulaire (9,11), notamment de forme cylindrique, suffisamment souple pour se conformer à ladite lumière naturelle, mais suffisamment rigide pour maintenir un passage artificiel dans ladite lumière, destiné à être placé dans ladite lumière naturelle, la paroi dudit tube comprenant un matériau biocompatible, relativement lisse et mou, tel qu'un caoutchouc silicone, au moins dans sa partie externe, caractérisée en ce que ladite prothèse comprend deux éléments tubulaires (9,11), tels que définis dans le préambule de la présente revendication, destinés à être disposés dans ladite lumière (1), respectivement de part et d'autre du sphincter (5), et attachés l'un à l'autre par un moyen de liaison (10), souple et déformable, destiné à être pris dans l'orifice du sphincter (5), et chaque dit élément tubulaire présentant une section extérieure sensiblement constante d'une extrémité à l'autre dudit élément.

2. Prothèse selon la revendication 1, caractérisée en ce que le moyen de liaison consiste en un manchon souple (10), dont la longueur est adaptée à celle du sphincter, et dont les deux extrémités sont reliées en continuité d'écoulement, respectivement avec les deux éléments tubulaires (9,11), la paroi dudit manchon souple étant susceptible de prendre deux conformations sous l'action du sphincter, à savoir une conformation d'obturation (Fig.4), par la contraction du sphincter (5), et une conformation d'écoulement (Fig.3), par le relâchement du sphincter.

3. Prothèse selon la revendication 2, caractérisée en ce que la paroi du manchon (10) est plus souple, notamment plus mince que la paroi de chaque élément tubulaire (9,11).

4. Prothèse selon la revendication 2, caractérisée en ce que la conformation d'obturation (Fig.4) correspond à une forme biconique ou en sablier.

5. Prothèse selon la revendication 2, caractérisée en ce que la conformation d'écoulement (Fig.3) correspond à une forme cylindrique.

6. Prothèse selon la revendication 2, caractérisée en ce que le manchon souple (10) est ajouré de manière distribuée selon son pourtour, notamment par des fentes ou fenêtres longitudinales (Fig.5).

7. Prothèse selon la revendication 2, caractérisée en ce que les éléments tubulaires (9,11) forment avec le manchon souple (10) dans sa conformation d'écoulement, un conduit de section intérieure sensiblement constante selon la direction longitudinale de la prothèse (8).

8. Prothèse selon la revendication 1, caractérisée en ce que le moyen de liaison (10) consiste en une pluralité de fils, attachés individuellement à leurs deux extrémités, respectivement aux deux éléments tubulaires (9,11)

9. Prothèse selon la revendication 1, caractérisée en ce que la paroi d'au moins un élément tubulaire (9,11) comporte une armature tubulaire, notamment une spirale métallique ou non, par exemple noyée dans le matériau du tube, ou apparente extérieurement.

10. Prothèse selon la revendication 9, caractérisée en ce que l'armature s'étend à partir du moyen de liaison (10), sur une portion de la longueur de l'élément tubulaire (9,11).

11. Prothèse selon la revendication 1, caractérisée en ce qu'au moins un élément tubulaire (9,11) est ajouré par des orifices distribués selon sa longueur.

12. Prothèse selon la revendication 1, caractérisée en ce que la surface extérieure de l'un (9) des éléments tubulaires présente une ou des encoches distribuées selon sa longueur, notamment une encoche en hélice, à la manière d'un filet de vis, ou des encoches en quinconce.

13. Prothèse selon la revendication 1, caractérisée en ce que l'extrémité de l'un (9) des éléments tubulaires, opposée au moyen de liaison (10), est transversalement obturée, notamment par un bout convexe, et présente au moins un orifice latéral de communication avec l'intérieur dudit élément.

14. Prothèse selon la revendication 1, caractérisée en ce qu'elle comprend un fil d'extraction (14) à son extrémité inférieure (15).

15. Prothèse selon la revendication 1, caractérisée en ce que le diamètre extérieur d'un élément tubulaire (11), dit inférieur, est supérieur au diamètre extérieur de l'autre élément tubulaire (9), dit supérieur.

16. Prothèse selon la revendication 1, caractérisée en ce qu'au moins un élément tubulaire (9,11) est revêtu à l'extérieur d'un produit thérapeutique.

17. Ensemble pour le traitement d'une lumière ou voie naturelle d'un corps humain ou animal, comprenant:
- une prothèse selon l'une quelconque des revendications 1 à 16
- un mandrin (60) semi-rigide, creux, dont la section extérieure est adaptée pour recevoir par emmanchement au moins une partie de ladite prothèse
- un poussoir rigide creux (63), dont la section intérieure est adaptée pour un emmanchement dudit poussoir sur le mandarin semi-rigide (60)
caractérisé en ce que :
- le mandrin (60) présente d'un côté une butée extérieure (61), et de l'autre côté, une extrémité fermée (62) contre laquelle l'extrémité obturée (9b) de la prothèse vient en butée dans sa position emmanchée, ladite extrémité fermée (62) présentant un oeil (62a) d'écoulement à l'intérieur dudit mandrin, pouvant venir en coïncidence avec l'orifice latéral (9c) de ladite prothèse, toujours dans sa position emmanchée ;
- le poussoir rigide creux (63), a une longueur adaptée pour servir, dans la position emmanchée dudit poussoir, d'entretoise entre la prothèse (8) emmanchée et la butée extérieure (61).

## Claims

1. Prosthesis (8) intended for the treatment of a natural lumen (1) or tract of a human or animal body through which a flow is effected on either side of a sphincter (5), said prosthesis comprising a tubular element (9, 11), in particular of cylindrical shape, which is sufficiently flexible to conform to said natural lumen, but sufficiently rigid to maintain an artificial passage in said lumen, and is intended to be placed in said natural lumen, the wall of said tube comprising a relatively smooth and soft biocompatible material, such as a silicone rubber, at least in its outer part, characterized in that said prosthesis comprises two tubular elements (9, 11), such as are defined in the preamble of the present claim, which are intended to be arranged in said lumen (1) on either side, respectively, of the sphincter (5), and are attached to each other by a flexible and deformable connection means (10) which is intended to be held in the orifice of the sphincter (5), and each said tubular element having an external cross-section which is substantially constant from one end to the other of said element.

2. Prosthesis according to Claim 1, characterized in that the connection means consists of a flexible sleeve (10), the length of which is adapted to that of the sphincter, and the two ends of which are connected, in continuity of flow, to the two tubular elements (9, 11) respectively, the wall of said flexible sleeve being capable of assuming two configurations under the action of the sphincter, namely a closed configuration (Fig. 4) upon contraction of the sphincter (5), and a flow configuration (Fig. 3) upon relaxation of the sphincter.

3. Prosthesis according to Claim 2, characterized in that the wall of the sleeve (10) is more flexible, and in particular thinner, than the wall of each tubular element (9, 11).

4. Prosthesis according to Claim 2, characterized in that the closed configuration (Fig. 4) corresponds to a biconical or hourglass shape.

5. Prosthesis according to Claim 2, characterized in that the flow configuration (Fig. 3) corresponds to a cylindrical shape.

6. Prosthesis according to Claim 2, characterized in that the flexible sleeve (10) is open-worked about its circumference, in particular by longitudinal slots or windows (Fig. 5).

7. Prosthesis according to Claim 2, characterized in that the tubular elements (9, 11) form, together with the flexible sleeve (10) in its flow configuration, a conduit having an internal cross-section which is substantially constant in the longitudinal direction of the prosthesis (8).

8. Prosthesis according to Claim 1, characterized in that the connection means (10) consists of a plurality of wires which are individually attached at their two ends to the two tubular elements (9, 11) respectively.

9. Prosthesis according to Claim 1, characterized in that the wall of at least one tubular element (9, 11) comprises a tubular reinforcement, in particular a metallic or non-metallic coil, for example embedded in the material of the tube, or externally exposed.

10. Prosthesis according to Claim 9, characterized in that the reinforcement extends from the connection means (10) over a part of the length of the tubular element (9, 11).

11. Prosthesis according to Claim 1, characterized in that at least one tubular element (9, 11) is open-worked with orifices distributed along its length.

12. Prosthesis according to Claim 1, characterized in that the outer surface of one (9) of the tubular elements has one or more notches distributed along its length, in particular a helical notch, in the manner of a screw thread, or staggered notches.

13. Prosthesis according to Claim 1, characterized in that the end of one (9) of the tubular elements, opposite the connection means (10), is closed transversely, in particular by a convex stump, and has at least one lateral orifice for communicating with the inside of said element.

14. Prosthesis according to Claim 1, characterized in that it comprises a removal wire (14) at its lower end (15).

15. Prosthesis according to Claim 1, characterized in that the external diameter of one tubular element (11), called the lower tubular element, is greater than the external diameter of the other tubular element (9), called the upper tubular element.

16. Prosthesis according to Claim 1, characterized in that at least one tubular element (9, 11) is coated on the outside with a therapeutic substance.

17. Assembly for treating a natural lumen or tract of a human or animal body, comprising:
- a prosthesis according to any one of Claims 1 to 16,
- a semi-rigid and hollow mandrel (60) whose external cross-section is adapted to receive at least a part of said prosthesis which is engaged onto it,
- a rigid and hollow pusher (63) whose internal cross-section is adapted for engagement of said pusher on the semi-rigid mandrel (60),
characterized in that:
- the mandrel (60) has, at one extremity, an outer limit stop (61), and, at the other extremity, a sealed end (62) against which the closed end (9b) of the prosthesis comes into abutment in its engaged position, said sealed end (62) having an eyelet (62a) for flow inside said mandrel, which eyelet (62a) can be brought into line with the lateral orifice (9c) of said prosthesis, again in its engaged position;
- the rigid and hollow pusher (63) has a length adapted to act, in the engaged position of said pusher, as a spacer between the engaged prosthesis (8) and the outer limit stop (61).

## Patentansprüche

1. Prothese (8) für die Behandlung eines natürlichen Lumens (1) der Weges eines menschlichen oder tierischen Körpers, durch den ein Ausfluß über einen Schließmuskel (5) hinweg erfolgt, wobei die Prothese ein rohrförmiges, insbesondere zylindrisches Element (9, 11) aufweist, das ausreichend biegsam ist, um sich an das natürliche Lumen anzupassen, das jedoch ausreichend steif ist, um einen künstlichen Durchgang in dem Lumen aufrecht zu erhalten, und das in das natürliche Lumen einsetzbar ist, wobei die Wand der Röhre zumindest in ihrem äußeren Bereich aus einem biokompatiblen, relativ glatten und weichen Material, wie einem Silikonkautschuk, besteht, dadurch gekennzeichnet, daß die Prothese zwei entsprechend dem Oberbegriff dieses Anspruches definierte rohrförmige Elemente (9, 11) aufweist, die beidseits des Schließmuskels (5) in dem Lumen (1) anordenbar sind, und die über ein biegsames und verformbares Verbindungsstück (10) miteinander verbunden sind, dar in der Öffnung des Schließmuskels (5) aufnehmbar ist, und wobei jedes der rohrförmigen Elemente, von dem einen zum anderen Ende dem jeweiligen Elementes, einen im wesentlichen konstanten Außenquerschnitt aufweist.

2. Prothese nach Anspruch 1, dadurch gekennzeichnet, daß das Verbindungselement aus einer biegsamen Muffe (10) besteht, deren Länge an den Schließmuskel angepaßt ist, und deren beide, Enden unter stetiger Ausflußmöglichkeit mit den beiden rohrförmigen Elementen (9, 11) verbunden sind, wobei die Wand der biegsamen Mutte unter der Wirkung des Schließmuskels zwei Formzustände einnehmen kann, und zwar eine Verschließform (Fig. 4) durch das Zusammenziehen des Schließmuskels (5) und eine Ausflußform (Fig. 3) beim Entspannen des Schließmuskels.

3. Prothese nach Anspruch 2, dadurch gekennzeichnet, daß die Wand der Muffe (10) biegsamer, insbesondere dünner als die Wand dar beiden rohrförmigen Elemente (9, 11) ist.

4. Prothese nach Anspruch 2, dadurch gekennzeichnet, daß die Verschließform (Fig. 4) der Form eines Doppelkonus bzw. der Form einer Sanduhr entspricht.

5. Prothese nach Anspruch 2, dadurch gekennzeichnet, daß die Ausflußform (Fig. 3) einer zylindrischen Form entspricht.

6. Prothese nach Anspruch 2, dadurch gekennzeichnet, daß die biegsame Muffe (10) insbesondere durch längs verlaufende Schlitze oder Fenster umfänglich verteilt durchbrochen ist (Fig. 5).

7. Prothese nach Anspruch 2, dadurch gekennzeichnet, daß die rohrförmigen Elemente (9, 11) zusammen mit der biegsamen Muffe (10) in deren Ausflußform einen Kanal mit einem längs der Längsrichtung der Prothese (8) im wesentlichen konstanten Innenquerschnitt bilden.

8. Prothese nach Anspruch 1, dadurch gekennzeichnet, daß das Verbindungselement (10) aus einer Vielzahl von Fäden gebildet wird, die mit ihren beiden Enden an den beiden rohrförmigen Elementen (9, 11) einzeln befestigt sind.

9. Prothese nach Anspruch 1, dadurch gekennzeichnet, daß die Wand zumindest eines rohrförmigen Elementes (9, 11) eine rohrförmige Verstärkung, insbesondere eine metallische oder nichtmetallische Spirale aufweist, die beispielsweise in das Material der Röhre eingebettet ist oder von außen sichtbar ist.

10. Prothese nach Anspruch 9, dadurch gekennzeichnet, daß sich die Verstärkung von dem Verbindungselement (10) ausgehend über einen Längenabschnitt des rohrförmigen Elementes (9, 11) erstreckt.

11. Prothese nach Anspruch 1, dadurch gekennzeichnet, daß zumindest ein rohrförmiges Element (9, 11) durch über dessen Länge verteilte Öffnungen durchbrochen ist.

12. Prothese nach Anspruch 1, dadurch gekennzeichnet, daß die Außenseite eines (9) der rohrförmigen Elemente eine oder mehrere der Länge nach verteilte Einkerbungen in der Art eines Schraubgewindes, insbesondere eine schraubenförmige Einkerbung aufweist oder gegeneinander versetzt angeordnete Einkerbungen aufweist.

13. Prothese nach Anspruch 1, dadurch gekennzeichnet, daß das dem Verbindungselement (10) entgegengesetzte Ende eines (9) der rohrförmigen Elemente, insbesondere durch eine konvexe Spitze, quer verschlossen ist und zumindest eine seitliche Öffnung zum Kommunizieren mit dem Innenraum dieses Elementes aufweist.

14. Prothese nach Anspruch 1, dadurch gekennzeichnet, daß sie an ihrem ausflußseitigen Ende (15) einen Faden (14) zum Herausziehen aufweist.

15. Prothese nach Anspruch 1, dadurch gekennzeichnet, daß der Außendurchmesser eines als abflußseitig bezeichneten rohrförmigen Elementes (11) größer als der Außendurchmesser des anderen, als zuflußseitig bezeichneten rohrförmigen Elementes (9) ist.

16. Prothese nach Anspruch 1, dadurch gekennzeichnet, daß zumindest ein rohrförmiges Element (9, 11) aut der Außenseite mit einem Arzneimittel versehen ist.

17. Zusammenbau zur Behandlung eines natürlichen Lumens oder eines matürlichen Weges eines menschlichen oder tierischen Körpers, mit
- einer Prothese nach einem der Ansprüche 1 - 16,
- einem hohlen halbstarren Dorn (60), dessen Außenquerschnitt so angepaßt ist, daß zumindest ein Abschnitt der Prothese auf diesen aufgeschoben werden kann,
- einer hohlen starren Vorschubstange (63), deren innenquerschnitt so angepaßt ist, daß sie auf den halbstarren Dorn (60) aufschiebbar ist,
dadurch gekennzeichnet, daß:
- der Dorn (60) auf einer Seite einen äußeren Anschlag (61) und auf der anderen Seite ein geschlossenes Ende (62) aufweist, gegen das das verschlossene Ende (9b) der Prothese in der aufgeschobenen Lage zur Anlage kommt, wobei das geschlossene Ende (62) ein Auge (62a) für ein Überfließen in den Innenraum des Dorns aufweist, das koinzident mit der seitlichen Öffnung (9c) der Prothese in deren aufgeschobenen Lage zusammenfällt; und daß
- die hohle starre Vorschubslange (63) eine solche Länge aufweist, daß sie in der vorgeschobenen Lage als Zwischenstück zwischen der aufgeschobenen Prothese (8) und dem äußeren Anschlag (61) dient.
